# EUROPEAN PATENT SPECIFICATION

(11) **EP 2 442 852 B1**
(45) Date of publication and mention of the grant of the patent: **01.05.2013**
(21) Application number: 10725726.3
(22) Date of filing: 18.06.2010
(51) Int. Cl.: A61M 5/28, A61M 5/14, A61M 5/148

(54) **INJECTION ARRANGEMENT FOR A FLOWABLE DRUG**
EINSPRITZANORDNUNG FÜR EIN FLIESSFÄHIGES ARZNEIMITTEL
Agencement d'injection pour médicament fluide

(30) Priority: 19.06.2009 EP 09008046
(43) Date of publication of application: 25.04.2012
(73) Proprietor: Sanofi-Aventis Deutschland GmbH, 65929 Frankfurt (DE)
(72) Inventor: BASSO, Nils, 65926 Frankfurt am Main (DE); NAGEL, Thomas, 01737 Tharandt (DE); RICHTER, René, 01737 Tharandt (DE); WITT, Robert, 01159 Dresden (DE)
(86) International application number: PCT/EP2010/058615
(87) International publication number: WO 2010/146149

(56) References cited:
- EP-A- 1 125 593
- EP-B- 1 450 882
- US-A- 2 757 669
- US-A- 5 656 033
- US-A1- 2008 275 590

## Description

The invention refers to an injection arrangement for delivering a flowable drug.

Medicament delivery devices in general comprise a reservoir for the medicament and means for displacing the medicament from the reservoir, usually in the shape of a plunger.

Medicament delivery devices of this type are restricted to a generally cylindrical form of the reservoir. Furthermore the device with the filled reservoir is about twice as long as the reservoir alone due to the length of the plunger.

US 5,527,288 discloses a reservoir in the form of an expansible-contractible chamber arranged within a housing member, the interior of the housing including a flexible liquid-impermeable membrane defining a first expansible-contractible chamber between it and a first non- deformable section of the housing, and a second expansible-contractible chamber defined between it and a second non-deformable section of the housing. The first chamber serves as a reservoir for receiving the drug to be delivered, whereas the second chamber typically serves as a gas-pumping chamber for controlling the delivery of the drug from the reservoir.

EP 1 450 882 B1 discloses a portable delivery device, pressurised by a pumped liquid and a cartridge therefore. The cartridge comprises a variable-volume drug reservoir having an outlet means and being adapted for containing a predefined volume of a flowable drug.

It is an object of the invention to provide an improved injection arrangement for delivering a flowable drug.

The object is achieved by an injection arrangement according to claim 1.

Advantageous embodiments are given in the dependent claims.

As compared to conventional injection arrangements the injection arrangement according to the invention avoids the friction associated with bungs and plungers. Furthermore the dead volume is considerably smaller since the volume inside the deformable bag can virtually be reduced to zero whereas in conventional injection arrangements there is usually some residual drug even with after fully advancing the bung due to manufacturing tolerances. Except for the cartridge the injection arrangement according to the invention is reusable since the flowable drug does not get in contact with the pump or the reservoir. The cartridge may easily be replaced.

The injection arrangement can either be a stationary or a portable device such as an Insulin pen.

Preferably the pump may be motor driven.

The outlet can comprise a hollow needle or a jet nozzle.

The displacement fluid is a liquid, for example oil and/or water. The liquid may have a low viscosity in order to reduce friction in narrow fluid passages. As liquids are almost incompressible, a high dosing accuracy of the flowable drug or medicament may be achieved.

In a preferred embodiment the deformable bag is attached at an end of the rigid outer casing opposite the outlet in order to ensure a precise folding of the deformable bag. E.g. in an elongate rigid outer casing the deformable bag is held at a front end in the region of the outlet where it is sealed against the rigid outer casing and at a back end opposite the outlet.

The deformable bag may be inserted into the rigid outer casing through the open outlet end of the rigid outer casing. The deformable bag may be attached to a closure member for sealing the open outlet end, wherein the closure member may have a fluid passage for allowing fluid communication between the deformable bag and the outlet.

The closure member may be gluod or welded or crimped onto the open outlet end in order to seal the interior of the rigid outer casing.

The deformable bag may comprise silicone or another flexible material.

In an example embodiment, the pump is a piston pump. The pump may comprise an electric motor and a transmission to translate the rotational movement of the motor into a translational movement of the piston. The pump may further comprise a gear to adapt the number of rotations of the motor to a defined displacement of the piston. By using a piston pump, the amount of displacement fluid, and thus the amount of the drug or medicament, may be proportional to the number of piston movements. The number of piston movements may further relate to the number of motor rotations. Thus, based at least in part on the number of motor rotations and / or piston movements, the amount of the displacement fluid pumped from the reservoir into the rigid outer casing may be determined. Therefore, by controlling and / or monitoring the number of motor rotations, or by controlling and / or monitoring the number of piston movements, the amount of expelled medicament can be determined.

In a preferred embodiment a flux sensor is arranged between the pump and the rigid outer casing. This allows for setting and controlling a dose of the flowable drug to be injected by measuring the flux of the displacement fluid without having to get the flux sensor in contact with the drug. The volume of the displacement fluid pumped into the rigid outer casing is the same as the volume of the delivered drug as long as the displacement fluid is a liquid not a gas, since liquids are virtually incompressible as opposed to gases. However, a gas may also be employed as the displacement fluid.

The reservoir may be integrated with the pump in a pump module. The pump module may be reused since it does not get in contact with the drug. The reservoir may be refillable in order to be reused. In this case the container cartridge is removed from the injection arrangement after use with displacement fluid still inside the rigid outer casing. In another embodiment the displacement fluid may be pumped back from the rigid outer casing into the reservoir after injection so the reservoir does not need to be refilled.

The injection arrangement may be operated in an inverse mode by pumping the displacement liquid out of the rigid outer casing into the reservoir thereby creating a vacuum in the deformable bag so the flowable drug or another flowable substance, e.g. blood may be sucked through the outlet into the deformable bag.

The injection arrangement may be advantageously used for delivering a flowable drug of the group Insulin, Heparin, Lovenox, human growth hormones, peptide hormones, analgetics and vaccines.

Further scope of applicability of the present invention will become apparent from the detailed description given hereinafter. However, it should be understood that the detailed description and specific examples, while indicating preferred embodiments of the invention, are given by way of Illustration only, since various changes and modifications within the scope of the invention will become apparent to those skilled in the art from this detailed description.

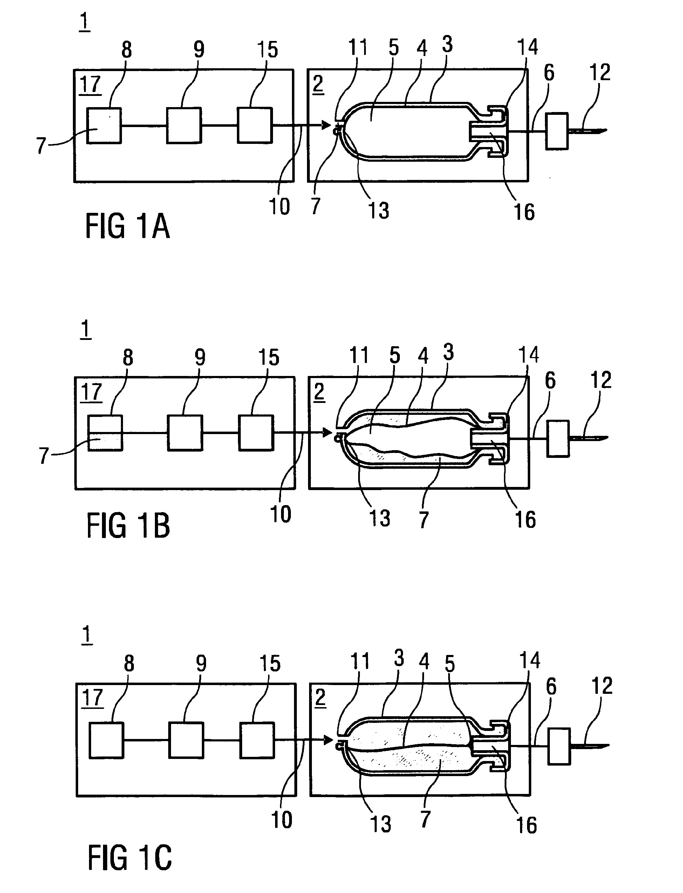

- Figure 1: is a schematic view of an injection arrangement with a container cartridge shown in a longitudinal section.

**Figure 1** shows a schematic view of an injection arrangement 1 with a replaceable container cartridge 2 shown in a longitudinal section. The container cartridge 2 comprises a rigid outer casing 3 and a deformable bag 4 arranged inside and sealed against the rigid outer casing 3. The deformable bag 4 holds a flowable drug 5 and is in fluid communication with an outlet 6 for delivering the flowable drug 5 to a human or an animal. The rigid outer casing 3 is fillable by a displacement fluid 7 for displacing the deformable bag 4 and thus the flowable drug 5 inside. The injection arrangement 1 further comprises a reservoir 8 for the displacement fluid 7 and a pump 9 for pumping the displacement fluid 7 from the reservoir 8 into the rigid outer casing 3 and/or from the rigid outer casing 3 into the reservoir 8 via a fluid channel 10 connected to the rigid outer casing 3 at an opening 11 thereof.

Figure 1a shows the injection arrangement 1 in an initial state, where the deformable bag 4 with the flowable drug 5 essentially fills the entire volume of the rigid outer casing 3.

Figure 1 b shows the injection arrangement 1 in a state with the rigid outer casing 3 partially filled by the displacement fluid 7 thus squeezing or displacing the deformable bag 4 and the flowable drug 5 inside and forcing the flowable drug 5 out of the outlet 6 in order to inject it into a human or an animal. The injection may be stopped at this point if the required dose of the drug 5 has been injected without emptying the whole content of the deformable bag 4.

However, as shown in figure 1 c, the whole content of the deformable bag 4 may be forced out of the outlet by entirely filling the rigid outer casing 3 with the displacement fluid 7 thereby fully squeezing the deformable bag 4.

The injection arrangement 1 can either be a stationary or a portable device such as an Insulin pen.

Preferably the pump 9 may be motor driven.

The outlet 6 can comprise a hollow needle 12 or a jet nozzle (not shown).

The displacement fluid 7 may be a liquid with a low viscosity such as oil and/or water. A gas may also be employed as the displacement fluid 7.

The deformable bag 4 is attached at an end of the rigid outer casing 3 opposite the outlet 6. In an elongate rigid outer casing 3 as shown in the figures the deformable bag 4 is held at a front end in the region of the outlet 6 where it is sealed against the rigid outer casing 3 and at a back end 13 opposite the outlet 6.

The deformable bag 4 may be inserted into the rigid outer casing 3 through the open outlet/front end of the rigid outer casing 3. The deformable bag 4 may be attached to a closure member 14 for sealing the open outlet/front end, wherein the closure member 14 may have a fluid passage 16 for allowing fluid communication between the deformable bag 4 and the outlet 6.

The closure member 14 may be glued or welded or crimped onto the open outlet/front end in order to seal the interior of the rigid outer casing 3.

The deformable bag 4 may comprise silicone or another flexible material.

A flux sensor 16 may be arranged between the pump 9 and the rigid outer casing 3. This allows for setting and controlling a dose of the flowable drug 5 to be injected by measuring the flux of the displacement fluid 7.

By measuring the amount of displacement fluid 7 with the flux sensor 15, the amount of drug or medicament 5 which is expelled may be determined without direct contact of the flux sensor 15 with the drug or medicament.

The reservoir 8 may be integrated with the pump 9 in a pump module 17. The pump module 17 may be reused. The reservoir 8 may be refillable. In this case the container cartridge 2 is removed from the injection arrangement 1 after use with the displacement fluid 7 still inside the rigid outer casing 3. In another embodiment the displacement fluid 7 may be pumped back from the rigid outer casing 3 into the reservoir 8 after injection so the reservoir 8 does not need to be refilled.

The injection arrangement 1 may be operated in an inverse mode by pumping the displacement liquid 7 out of the rigid outer casing 3 into the reservoir 8 thereby creating a vacuum in the deformable bag 4 so the flowable drug 5 or another flowable substance, e.g. blood may be sucked through the outlet 6 into the deformable bag 4. In this inverse mode the initial state is essentially the one shown in figure 1c,

The injection arrangement 1 may be advantageously used for delivering a flowable drug 5 of the group Insulin, Heparin, Lovenox, human growth hormones, peptide hormones, analgetics and vaccines.

The term "drug" or "medicament", as used herein, may also mean a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutical active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin, Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(830) human insulin; B29-N-palmitoyl-des(B30) human insulin; B29-N-myristoyl human insulin; B29-N-palmitoyl human Insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29Lys1330 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(830) human insulin and B29-N-(w-carboxyhepta-decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(02)25, Asp28] Exendin-4(1-39)-NH2.
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium,

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.). Mark Publishing Company, Easton, Pa., U.S.A., 1985 and in Encyclopedia of Pharmaceutical Technology.

Pharmaceutically acceptable solvates are for example hydrates.

### LIST OF REFERENCES

- 1: injection arrangement
- 2: container cartridge
- 3: rigid outer casing
- 4: deformable bag
- 5: flowable drug
- 6: outlet
- 7: displacement fluid
- 8: reservoir
- 9: pump
- 10: fluid channel
- 11: opening
- 12: hollow needle
- 13: back end
- 14: closure member
- 15: flux sensor
- 16: fluid passage
- 17: pump module

## Claims

1. Injection arrangement (1) comprising a replaceable container cartridge (2) for a flowable drug (5), the container cartridge (2) comprising a deformable bag (4) arranged inside and sealed against a rigid outer casing (3), the deformable bag (4) being arranged for holding the flowable drug (5) and in fluid communication with an outlet (6), the rigid outer casing (3) fillable by a displacement fluid (7) for displacing the deformable bag (4), the injection arrangement (1) further comprising a reservoir (8) holding the displacement fluid (7) and a pump (9) for pumping the displacement fluid (7) from the reservoir (8) into the rigid outer casing (3) and/or from the rigid outer casing (3) into the reservoir (8) via a fluid channel (10), wherein the displacement fluid (7) is a liquid and wherein the injection arrangement comprises a flux sensor, **characterized in that** the flux sensor (15) is arranged between the pump (9) and the rigid outer casing (3).

2. injection arrangement (1) according to claim 1, **characterized in that** the outlet (6) comprises a hollow needle (12).

3. injection arrangement (1) according to claim 1, **characterized in that** the outlet (6) comprises a jet nozzle.

4. injection arrangement (1) according to one of the preceding claims, **characterized in that** the displacement fluid (7) comprises oil and/or water.

5. Injection arrangement (1) according to one of the preceding claims, **characterized in that** the deformable bag (4) is attached at an end of the rigid outer (3) casing opposite the outlet (6).

6. injection arrangement (1) according to one of the preceding claims, **characterized in that** the deformable bag (4) is inserted into the rigid outer casing (3) through an open outlet end of the rigid outer casing (3), wherein the deformable bag (4) is attached to a closure member (14) for sealing the open outlet end, the closure member (14) having a fluid passage (16) for allowing fluid communication between the deformable bag (4) and the outlet (6).

7. injection arrangement (1) according to claim 6, **characterized in that** the closure member (14) is glued or welded or crimped onto the open outlet end.

8. injection arrangement (1) according to one of the preceding claims, **characterized in that** the deformable bag (4) comprises silicone.

9. Injection arrangement (1) according to one of the preceding claims, **characterized in that** the pump (9) is a piston pump comprising an electric motor wherein an amount of the displacement fluid (7) pumped from the reservoir (8) into the rigid outer casing (3) may be determined based at least in part on the number of motor rotations.

10. Injection arrangement (1) according to one of the preceding claims, **characterized in that** the reservoir (8) is integrated with the pump (9) in a pump module (17).

11. Injection arrangement (1) according to one of the preceding claims, **characterized in that** the reservoir (8) is refillable.

12. Use of an injection arrangement (1) according to one of the preceding claims for delivering a flowable drug of the group Insulin, Heparin, Lovenox, human growth hormones, peptide hormones, analgetics and vaccines.

13. Method for operating an injection arrangement (1), the injection arrangement comprising a replaceable container cartridge (2) for a flowable drug (5), the container cartridge (2) comprising a deformable bag (4) arranged inside and sealed against a rigid outer casing (3), the deformable bag (4) being arranged for holding the flowable drug (5) and in fluid communication with an outlet (6), the rigid outer casing (3) fillable by a displacement fluid (7) for displacing the deformable bag (4), the injection arrangement (1) further comprising a reservoir (8) for the displacement fluid (7) and a pump (9), the method comprising the step of pumping the displacement fluid (7) from the reservoir (8) into the rigid outer casing (3) and/or from the rigid outer casing (3) into the reservoir (8) via a fluid channel (10), wherein the displacement fluid (7) used is a liquid, wherein a flux sensor (15) is used for setting and controlling a dose of the flowable drug (5) to be injected, **characterized in that** the flux sensor (15) is arranged between the pump (9) and the rigid outer casing (3) for measuring the flux of the displacement fluid (7).

14. Method according to claim 13, **characterized in that** the displacement fluid (7) comprises oil and/or water.

## Patentansprüche

1. Einspritzanordnung (1) mit einer auswechselbaren Behälterkassette (2) für ein fließfähiges Arzneimittel (5), wobei die Behälterkassette (2) einen verformbaren Beutel (4) umfasst, der in einem starren Außengehäuse (3) angeordnete und dagegen abgedichtet ist, wobei der verformbare Beutel (4) zum Halten des fließfähigen Arzneimittels (5) und in Flüssigkeitsverbindung mit einem Auslass (6) angeordnet ist, wobei das starre Außengehäuse (3) mit einer Verdrängungsflüssigkeit (7) zum Verdrängen des verformbaren Beutels (4) gefüllt werden kann, wobei die Einspritzanordnung (1) ferner ein Reservoir (8), das die Verdrängungsflüssigkeit (7) enthält, und eine Pumpe (9) zum Pumpen der Verdrängungsflüssigkeit (7) aus dem Reservoir (8) in das starre Außengehäuse (3) und/oder aus dem starren Außengehäuse (3) in das Reservoir (8) über einen Flüssigkeitskanal (10) umfasst, worin die Verdrängungsflüssigkeit (7) eine Flüssigkeit ist und worin die Einspritzanordnung einen Flusssensor umfasst, **dadurch gekennzeichnet, dass** der Flusssensor (15) zwischen der Pumpe (9) und dem starren Außengehäuse (3) angeordnet ist.

2. Einspritzanordnung (1) nach Anspruch 1, **dadurch gekennzeichnet, dass** der Auslass (6) einen Hohlnadel (12) umfasst.

3. Einspritzanordnung (1) nach Anspruch 1, **dadurch gekennzeichnet, dass** der Auslass (6) eine Strahldüse umfasst.

4. Einspritzanordnung (1) nach einem der vorhergehenden Ansprüche, **dadurch gekennzeichnet, dass** die Verdrängungsflüssigkeit (7) Öl und/oder Wasser umfasst.

5. Einspritzanordnung (1) nach einem der vorhergehenden Ansprüche, **dadurch gekennzeichnet, dass** der verformbare Beutel (4) an einem Ende des starren Außengehäuses (3) gegenüber dem Auslass (6) befestigt ist.

6. Einspritzanordnung (1) nach einem der vorhergehenden Ansprüche, **dadurch gekennzeichnet, dass** der verformbare Beutel (4) in das starre Außengehäuse (3) durch ein offenes Auslassende des starren Außengehäuses (3) eingeführt ist, worin der verformbare Beutel (4) an einem Verschlusselement (14) zum Abdichten des offenen Auslassendes befestigt ist, wobei das Verschlusselement (14) einen Flüssigkeitsdurchgang (16) zur Flüssigkeitsverbindung zwischen dem verformbaren Beutel (4) und dem Auslass (6) aufweist.

7. Einspritzanordnung (1) nach Anspruch 6, **dadurch gekennzeichnet, dass** das Verschlusselement (14) auf das offene Auslassende geklebt oder geschweißt oder gequetscht ist.

8. Einspritzanordnung (1) nach einem der vorhergehenden Ansprüche, **dadurch gekennzeichnet, dass** der verformbare Beutel (4) Silikon umfasst.

9. Einspritzanordnung (1) nach einem der vorhergehenden Ansprüche, **dadurch gekennzeichnet, dass** die Pumpe (9) eine Kolbenpumpe ist, die einen Elektromotor umfasst, worin eine Menge der Verdrängungsflüssigkeit (7), die aus dem Reservoir (8) in das starre Außengehäuse (3) gepumpt wird, zumindest teilweise auf Basis der Anzahl von Motorumdrehungen ermittelt werden kann.

10. Einspritzanordnung (1) nach einem der vorhergehenden Ansprüche, **dadurch gekennzeichnet, dass** das Reservoir (8) mit der Pumpe (9) in einem Pumpenmodul (17) integriert ist.

11. Einspritzanordnung (1) nach einem der vorhergehenden Ansprüche, **dadurch gekennzeichnet, dass** das Reservoir (8) aufgefüllt werden kann.

12. Verwendung einer Einspritzanordnung (1) nach einem der vorhergehenden Ansprüche zur Abgabe eines fließfähigen Arzneimittels aus der Gruppe von Insulin, Heparin, Lovenox, humane Wachstumshormone, Peptidhormone, Analgetika und Impfstoffe.

13. Verfahren zur Bedienung einer Einspritzanordnung (1), wobei die Einspritzanordnung eine auswechselbare Behälterkassette (2) für ein fließfähiges Arzneimittel (5) umfasst, wobei die Behälterkassette (2) einen verformbaren Beutel (4) umfasst, der in einem starren Außengehäuse (3) angeordnete und dagegen abgedichtet ist, wobei der verformbare Beutel (4) zum Halten des fließfähigen Arzneimittels (5) und in Flüssigkeitsverbindung mit einem Auslass (6) angeordnet ist, wobei das starre Außengehäuse (3) mit einer Verdrängungsflüssigkeit (7) zum Verdrängen des verformbaren Beutels (4) gefüllt werden kann, wobei die Einspritzanordnung (1) ferner ein Reservoir (8), das die Verdrängungsflüssigkeit (7) enthält, und eine Pumpe (9) umfasst, wobei das Verfahren die Schritte des Pumpens der Verdrängungsflüssigkeit (7) aus dem Reservoir (8) in das starre Außengehäuse (3) und/oder aus dem starren Außengehäuse (3) in das Reservoir (8) über einen Flüssigkeitskanal (10) umfasst, worin die Verdrängungsflüssigkeit (7) eine Flüssigkeit ist und worin ein Flusssensor (15) zur Einstellung und Kontrolle einer Dosis des zu injizierenden flüssigen Arzneimittels (5) umfasst, **dadurch gekennzeichnet, dass** der Flusssensor (15) zwischen der Pumpe (9) und dem starren Außengehäuse (3) zur Messung des Flusses der Verdrängungsflüssigkeit (7) angeordnet ist.

14. Verfahren nach Anspruch 13, **dadurch gekennzeichnet, dass** die Verdrängungsflüssigkeit (7) Öl und/oder Wasser umfasst.

## Revendications

1. Agencement d'injection (1) comprenant une cartouche de récipient remplaçable (2) pour un médicament fluide (5), la cartouche de récipient (2) comprenant un sac déformable (4) agencé à l'intérieur de et scellé contre une enveloppe extérieure rigide (3), le sac déformable (4) étant agencé de façon à contenir le médicament fluide (5) et étant en communication fluide avec une sortie (6), l'enveloppe extérieure rigide (3) pouvant être remplie par un fluide de déplacement (7) pour déplacer le sac déformable (4), l'agencement d'injection (1) comprenant en outre un réservoir (8) contenant le fluide de déplacement (7) et une pompe (9) pour pomper le fluide de déplacement (7) du réservoir (8) dans l'enveloppe extérieure rigide (3) et/ou de l'enveloppe extérieure rigide (3) dans le réservoir (8) via un canal de fluide (10), dans lequel le fluide de déplacement (7) est un liquide et dans lequel l'agencement d'injection comprend un détecteur de flux, **caractérisé en ce que** le détecteur de flux (15) est agencé entre la pompe (9) et l'enveloppe extérieure rigide (3).

2. Agencement d'injection (1) selon la revendication 1, **caractérisé en ce que** la sortie (6) comprend une aiguille creuse (12).

3. Agencement d'injection (1) selon la revendication 1, **caractérisé en ce que** la sortie (6) comporte une tuyère d'éjection.

4. Agencement d'injection (1) selon l'une quelconque des revendications précédentes, **caractérisé en ce que** le fluide de déplacement (7) comprend de l'huile et/ou de l'eau.

5. Agencement d'injection (1) selon l'une quelconque des revendications précédentes, **caractérisé en ce que** le sac déformable (4) est attaché à une extrémité de l'enveloppe extérieure rigide (3) en face de la sortie (6).

6. Agencement d'injection (1) selon l'une quelconque des revendications précédentes, **caractérisé en ce que** le sac déformable (4) est inséré dans l'enveloppe extérieure rigide (3) à travers une extrémité de sortie ouverte de l'enveloppe extérieure rigide (3), dans lequel le sac déformable (4) est attaché à un élément de fermeture (14) pour obturer l'extrémité de sortie ouverte, l'élément de fermeture (14) comportant un passage de fluide (16) pour permettre une communication fluide entre le sac déformable (4) et la sortie (6).

7. Agencement d'injection (1) selon la revendication 6, **caractérisé en ce que** l'élément de fermeture (14) est collé ou soudé ou serti sur l'extrémité de sortie ouverte.

8. Agencement d'injection (1) selon l'une quelconque des revendications précédentes, **caractérisé en ce que** le sac déformable (4) contient du silicone.

9. Agencement d'injection (1) selon l'une quelconque des revendications précédentes, **caractérisé en ce que** la pompe (9) est une pompe à piston comportant un moteur électrique, dans lequel une quantité du fluide de déplacement (7) pompée hors du réservoir (8) dans l'enveloppe extérieure rigide (3) peut être déterminée au moins en partie sur la base du nombre de rotations du moteur.

10. Agencement d'injection (1) selon l'une quelconque des revendications précédentes, **caractérisé en ce que** le réservoir (8) est intégré avec la pompe (9) dans un module de pompe (17).

11. Agencement d'injection (1) selon l'une quelconque des revendications précédentes, **caractérisé en ce que** le réservoir (8) peut être de nouveau rempli.

12. Utilisation d'un agencement d'injection (1) selon l'une quelconque des revendications précédentes pour délivrer un médicament fluide du groupe de l'insuline, de l'héparine, du lovenox, des hormones de croissance humaines, des hormones peptidiques, des analgésiques et des vaccins.

13. Procédé pour utiliser un agencement d'injection (1), l'agencement d'injection comprenant une cartouche de récipient remplaçable (2) pour un médicament fluide (5), la cartouche de récipient (2) comprenant un sac déformable (4) agencé à l'intérieur de et scellé contre une enveloppe extérieure rigide (3), le sac déformable (4) étant agencé de façon à contenir le médicament fluide (5) et étant en communication fluide avec une sortie (6), l'enveloppe extérieure rigide (3) pouvant être remplie par un fluide de déplacement (7) pour déplacer le sac déformable (4), l'agencement d'injection (1) comprenant en outre un réservoir (8) pour le fluide de déplacement (7) et une pompe (9), le procédé comprenant l'étape de pomper le fluide de déplacement (7) du réservoir (8) dans l'enveloppe extérieure rigide (3) et/ou de l'enveloppe extérieure rigide (3) dans le réservoir (8) via un canal de fluide (10), dans lequel le fluide de déplacement utilisé (7) est un liquide, dans lequel on utilise un détecteur de flux (15) pour régler et commander une dose du médicament fluide (5) à injecter, **caractérisé en ce que** le détecteur de flux (15) est agencé entre la pompe (9) et l'enveloppe extérieure rigide (3) pour mesurer le flux du fluide de déplacement (7).

14. Procédé selon la revendication 13, **caractérisé en ce que** le fluide de déplacement (7) comprend de l'huile et/ou de l'eau.
